# EUROPEAN PATENT SPECIFICATION

(11) **EP 1 387 620 B1**
(45) Date of publication and mention of the grant of the patent: **04.11.2009**
(21) Application number: 02724329.4
(22) Date of filing: 08.05.2002
(51) Int. Cl.: A23K 1/00, A23L 1/09

(54) **METHOD FOR PRODUCING A NUTRITION ADDITIVE, AN ADDITIVE AND ITS USE**
VERFAHREN ZUR HERSTELLUNG EINES NAHRUNGSMITTELZUSATZES, EIN ZUSATZ UND SEINE VERWENDUNG
PROCEDE DE PRODUCTION D'UN ADDITIF ALIMENTAIRE, ADDITIF ALIMENTAIRE ET UTILISATION CORRESPONDANTE

(30) Priority: 14.05.2001 FI 20011008
(43) Date of publication of application: 11.02.2004
(73) Proprietor: SUOMEN REHU OY, 00500 Helsinki (FI)
(72) Inventor: VUORENMAA, Juhani, FIN-33400 Tampere (FI); RAUTONEN, Nina, FIN-02170 Espoo (FI)
(74) Representative: Partio, Erja
(86) International application number: PCT/FI2002/000393
(87) International publication number: WO 2002/091850

(56) References cited:
- EP-A1- 0 549 478
- EP-A1- 0 920 812
- WO-A1-96/38057
- WO-A1-98/27829

## Description

The present invention relates to a method as defined in the preamble of claim 1 for producing a nutrition additive. The invention also concerns a nutrition additive, its use and a preparation containing the additive.

Intestinal microbial balance is a prerequisite for the health and well-being as well as productivity of animals. Disturbances of this balance appear as diarrhea and other intestinal health problems, and they may even lead to the death of the animal.

Single-stomach animals are protected against the influence of detrimental microbes by adding various antibiotics and chemotherapeutics inhibiting microbial growth to the fodder used to feed the animal. Also, probiotic products, such as various microbes, acids and yeasts, may be added to fodders to maintain intestinal balance and to avoid the use of antibiotics. There are also fodder mixtures with oligosaccharides added to them to inhibit the growth of harmful microbes or to assist the growth of useful microbes. Further, patent application FI 965192 discloses a yeast hydrolysate containing oligosaccharides and/or polysaccharides and its use for the prevention of intestinal diseases.

The use of antibiotics involves the problem of development of microbial strains immune to antibiotics and consequent health risks to humans. A problem with probiotic products is their varying and generally low efficiency; moreover, the costs of their use are fairly high. A problem with fodder containing pure oligosaccharides and the previously known fodder containing oligosaccharides and/or polysaccharides is likewise a varying and generally too weak an influence of oligosaccharides and/or polysaccharides in the prevention of intestinal diseases. In addition, the price of pure oligosaccharides is high.

The object of the present invention is to eliminate the above-mentioned drawbacks.

A specific object of the invention is to disclose a method for producing a nutrition additive that can be used to exert a more effective influence on intestinal microbes so as to promote the health and/or growth of animals.

A further object of the invention is to disclose an effective nutrition additive of uniform quality that can be used to reduce intestinal diseases of animals in a cost-saving manner.

A further object of the invention is to disclose the use of a new additive produced according to the invention and a preparation containing said additive.

As for the features characteristic of the invention, reference is made to the claims.

In the method of the invention for producing a nutrition additive, brewery yeast is filtered and processed in such manner that its cellular structure is altered and the effective concentration of oligo-and/or polysaccharides, betaglucane and/or proteins on the surface of the cellular structures is increased, i.e. the cellular structure is broken down, to release oligo- and/or polysaccharides, betaglucane and/or proteins for utilization for the prevention of intestinal diseases.

The invention also discloses products manufactured by this method, their use and preparations containing additives in accordance with the claims.

The invention is based on research work in which nutrition additives were investigated, and in which the unexpected observation was made that, by first filtering brewery yeast and processing it further hydrolytically so that the cellular structure of the brewery yeast is broken down, a product is obtained that, when given to an animal together with fodder, reduces intestinal diseases of the animal considerably more effectively than a product obtained by merely treating brewery yeast by a hydrolytic process.

The mechanism of influence of the product thus obtained in the prevention of intestinal diseases is based on the fact that its use in connection with fodders inhibits microbial adhesion to the bowel, which means that oligo- and/or polysaccharides and/or proteins released in connection with the filtering of brewery yeast and breakdown of cellular structure function in a manner analogous to receptors of harmful microbes, such as E.coli, in the bowel, weakening the ability of microbes to adhere to the bowel wall.

Further, oligo- and/or polysaccharides, betaglucane, proteins, nucleotides, peptides and/or other substances released in connection with the aforesaid breakdown of the cellular structure of brewery yeast are useful in respect of regulation of intestinal microbial population.

The breakdown products obtained via the filtering and hydrolysis process have an influence on the animal's immune response, i.e. certain components of brewery yeast, e.g. the sugar structures of yeast that contain phosphor, can improve the immune response of animals, thus inhibiting intestinal diseases. The production method additionally has an effect on the type and intensity of the immune response.

Brewery yeast is produced as a by-product of beer industry. Normally, the brewery yeast mixture is passed after beer production into storage containers, where brewery yeast is deposited in the bottom part of the container. After this, the beer remaining on the surface is separated. The dry matter content of brewery yeast thus obtained usually varies in the range of 7 - 13 w-%.

In the method of the invention, the brewery yeast deposited on the bottom of the container is filtered mechanically and/or pneumatically. The brewery yeast may be filtered by any known filtering method. To prevent clogging of the filters, e.g. filter plates, it is possible to use a vibrator, e.g. a micro-vibrator at a high frequency, and/or some other corresponding technique preventing clogging. The filter density is selected on the basis of the particle size of the yeast.

The dry matter content of brewery yeast filtered according to the invention is at least 15 w-%, preferably 18 - 20 w-%.

In the brewery yeast used, the oligo- and/or polysaccharides, betaglucane, proteins, such as manno-protein, are bonded in the cellular structures of the raw material.

In the method of the invention, the cellular structure of brewery yeast is broken down hydrolytically using an acid and/or an alkali, via autolysis and/or enzymatically. Besides hydrolytic processing, heat treatment and/or detergent treatment of brewery yeast are/is also possible, and/or a treatment breaking down the cellular structure of the raw material may be employed, e.g. by subjecting the cellular structure to a mechanical, hydrostatic and/or pneumatic force.

The acids used in hydrolysis may be e.g. conventional mineral acids, such as hydrochloric acid, sulphuric acid, phosphoric acid, nitric acid and so on, as well as strong organic acids, e.g. formic acid, acetic acid, propionic acid and so on. The pH range used in hydrolysis may be below 4, e.g. about 2 - 4. In alkaline hydrolysis, the alkalis used may be e.g. conventional alkaline hydroxides, such as sodium hydroxide, potassium hydroxide etc., ammonium hydroxide or other alkalis releasing oligo- and/or polysaccharides.

Enzymes usable in enzymatic hydrolysis include e.g. various proteases, e.g. acid or alkaline proteases. In the hydrolysis, it is also possible to use the enzyme contained in the brewery yeast itself, in which case hydrolysis occurs via autolysis. Further, the hydrolysis can be implemented using other added enzymes, proteases, ribonucleases and deaminases. In enzymatic processing it is also possible to use a combination of several enzymes, simultaneously and/or in succession, e.g. papaine, ribonuclease and/or deaminase. In general, the method may be implemented using enzymes presented in the specifications referred to below and/or other enzymes known in themselves and having the desired effect of breaking down the cellular structure of the raw material, together and/or separately, e.g. as described in the specifications referred to below.

In hydrolysis, the yeast may be heated to a temperature exceeding 40 °C, in autolysis and enzymatic hydrolysis e.g. to 40 - 65 °C and in acid and alkaline hydrolysis e.g. to 70 - 95 °C. The duration of the heating period may vary depending on the temperature, e.g. 1 - 12 h.

The insoluble and soluble fractions obtained via hydrolysis contain desired oligo- and/or polysaccharides, betaglucane and/or proteins.

Hydrolytic breakdown of yeasts is described in patent specifications and applications: FI 965192, WO 96/38057 and GB 1032687. These methods as well as other known methods can be used in connection with the present invention, the usable product being expressly the unfractionated product as such or the insoluble or soluble fraction.

The product manufactured by the method of the invention can be added to a fodder or foodstuff directly as such, in a moistened or dried form, and it can be generally treated in a desired manner.

The nutrition additive produced by the method of the invention can be used in fodders intended for single-stomach animals, e.g. swine, especially pigs, poultry, such as hens, broiler chickens and turkeys, calves, fur animals, such as foxes, and pets, such as dogs and cats, horses, especially foals, fishes and so on, to prevent intestinal diseases. The amount of nutrition additive used in fodders/nutrition of single-stomach animals may be 0.01 - 1.0 w-%, preferably 0.1 - 0.3 w-% of the total amount of fodder, calculated in terms of dry matter. The additive can be used together with fodder/nutrition or as such. Suitable use of the additive is such that the daily intake is 0.1 - 10 g/day, preferably 0.1 - 3 g/day.

The nutrition additive of the invention can also be used in human food, e.g. in food preparations for children or adults or as a preparation served separately to promote health, for balancing intestinal microbes and preventing intestinal diseases.

When added to animal fodder, the additive produced by the method of the invention is by about 25 - 30 % more effective in inhibiting the growth of harmful microbes and promoting the growth of useful microbes than a product made from unfiltered brewery yeast. At the same time, animal growth, utilization of fodder and overall production economy are improved. In addition, the useful constituents of yeast are preserved in the same product. Further, the invention allows cost-saving manufacture of the additive and preparation of the invention.

In addition, the use of fodder products according to the invention, i.e. natural fodder products in animal fodder provides a chance to stop using fodder antibiotics. The risk of development of microbial strains immune to antibiotics is diminished and the human health hazards caused by them are reduced.

The invention will now be described in detail with reference to the following examples of its embodiments.

### EXAMPLE 1

In an experiment, a nutrition additive according to the invention was produced from brewery yeast with a dry matter content of 9 w-%, obtained from beer industry.

The brewery yeast was filtered mechanically by means of a fine filter, by vibrating the filter plates with a micro-vibrator at a high frequency. The yeast was filtered to a dry matter content of 18 w-%. During the filtering, 3 - 4 % of the dry matter was removed with the liquid, and the dry matter yield of yeast was 50 - 60 % of the estimated quantity.

The filtered brewery yeast was hydrolyzed with an acid. In the hydrolysis, the pH of the yeast slurry was held at a value of 2 - 3 using a strong acid (4 h) and at a temperature of 70 - 85 °C. Next, the pH was increased to a value of 4 - 5, and the product obtained was cooled. The final product obtained can be used as such or it may be dried by known methods.

### EXAMPLE 2

In this experiment, the effect of a yeast hydrolysate as prepared in Example 1 on the adhesion of the E.coli bacterium to porcine mucous membranes on micro-titer plates was compared with the corresponding effect of a, yeast hydrolysate produced by the method disclosed in patent application FI 965192; the experiment is described in the publication Conway, P.L., (1990) Infection and Immunity, 58, 3178-3182. Presence of K88-specific receptors in porcine ileal mucus is age dependent.

The results are presented in Table 1.

**Table 1**

| | Adhesion of E.coli, % | | | |
|---|---|---|---|---|
| Quantity of test material in analysis % | Control | Hydrolysate, unfiltered yeast | Hydrolysate, filtered yeast | % filtered from unfiltered material |
| | 100 | | | |
| 0.16 | | 21.4 | 16.2 | 76 |
| 0.08 | | 45.8 | 32.4 | 71 |
| 0.016 | | 82.3 | 60.7 | 74 |

It was established from the results that the additive of the invention was more effective by about 25 - 30 % in inhibiting adhesion of E.coli than a product made from unfiltered brewery yeast.

### EXAMPLE 3

An experiment was carried out to investigate the effect of the yeast hydrolysate prepared in Example 1 and of a hydrolysate 2 according to the invention on intestinal immunity of rats. The immunity was determined by measuring the IgA content in the digestive tract and determining the proportions of immune cells from samples of intestinal tissue. The comparative determinations were made using a control and a betaglucane product.

In each test, 6 rats were used. Tissue samples were taken from the duodenum and ileum 28 days after the beginning of the feeding experiment. The samples were diluted and their IgA was determined by the new ELISA method, which is used to measure the immune response at bowel level. By earlier methods, immunity has been measured indirectly from cell cultures or blood samples. By the method now used, it is possible to measure both cell-mediated immunity and antibody-mediated immunity in the bowel and thus to directly measure the inhibiting effect of different products against intestinal diseases. In the determinations, monoclonal antibodies specific to immune cells of rats were used.

The product according to the invention (0.3 %) and betaglucane had no great effect on IgA content in the digestive tract. Both increased the IgA content as compared with the control. In smaller rations, the product of the invention increased the IgA content as compared with the control.

The frequencies of macrophages and CD8-positive cells (positive cells/0.5 villus) are presented in Table 2.

**Table 2**

| | Control | Betaglucane | Hydrolysate of Example 1 | Hydrolysate 2 |
|---|---|---|---|---|
| Macrophages | 8.2 | 5.8 | 15.7 | 13.0 |
| CD8+ | 8.5 | 13.0 | 19.5 | 15.0 |

It can be seen from Table 2 that the product of the invention caused a significant increase in the frequency of macrophage cells and a definite increase in the frequency of CD8-positive cells. The product of the invention stimulated locally in the digestive tract those types of immune response that have an importance especially in intracellular infections (viruses, parasites, and bacteria proliferating in cells), giving additional protection even against other infections besides those caused by E.coli, whereas the betaglucane product was more or less ineffective.

The invention is not limited to the examples of its embodiments described above; instead, variations of it are possible within the scope of the inventive idea defined in the claims.

## Claims

1. Method for producing a nutrition additive for use for the prevention of intestinal diseases, in which method brewery yeast is treated by a hydrolytic process, **characterized in that** brewery yeast is filtered and the filtered brewery yeast is treated hydrolytically so that the cellular structure breaks open and the effective concentration of oligo- and/or polysaccharides, betaglucane and/or proteins on the surface of the cellular structures is increased.

2. Method according to claim 1, **characterized in that** the brewery yeast is filtered mechanically.

3. Method according to claim 1 or 2, **characterized in that** the brewery yeast is filtered to a minimum dry matter content of 15 %, preferably to a dry matter content of 18 - 20 %.

4. Method according to any one of claims 1 - 3, **characterized in that** the filtered brewery yeast is treated with an acid and/or an alkali.

5. Method according to any one of claims 1 - 4**, characterized in that** the filtered brewery yeast is treated enzymatically.

6. Method according to any one of claims 1 - 5, **characterized in that** the filtered brewery yeast is treated with heat and/or mechanically, hydrostatically or pneumatically so that the cellular structure is broken down.

7. Method according to any one of claims 1 - 6, **characterized in that** the product obtained is used as such without fractionation.

8. Method according to any one of claims 1 - 7, **characterized in that** the oligo- and/or polysaccharide, betaglucane and/or protein product obtained is added to nourishment in an amount of 0.01 - 1.0 w-%, preferably 0.1 - 0.3 w-%, calculated in terms of dry matter.

9. Nutrition additive for the prevention of intestinal diseases, **characterized in that** the additive has been produced by treating filtered brewery yeast by a hydrolytic process so that the cellular structure breaks open and the effective concentration of oligo- and/or polysaccharides, betaglucane and/or proteins on the surface of the cellular structures is increased.

10. Additive according to claim 9, **characterized in that** the additive has been produced by mechanically filtering brewery yeast.

11. Additive according to claim 9 or 10, **characterized in that** the additive has been produced by treating the raw material with an acid and/or an alkali.

12. Additive according to any one of claims 9 - 11, **characterized in that** the additive has been produced by treating the raw material enzymatically.

13. Additive according to any one of claims 9 - 12, **characterized in that** the brewery yeast has been treated with heat and/or mechanically, hydrostatically or pneumatically so that the cellular structure has been broken down.

14. Use of a nutrition additive as defined in any one of claims 9 - 13 for preparation of nutrition intended for the prevention of intestinal diseases, the amount of the additive used being 0.01 - 1.0 w-%, preferably 0.1 - 0.3 w-% mixed in nutritive substances.

15. Preparation containing a nutrition additive and intended to be given to a creature to be fed, **characterized in that** the preparation contains a product as defined in any one of claims 10 - 13 in an amount of 0.01 - 1.0 w-%, preferably 0.1 - 0.3 w-% of the daily portion of foodstuff and/or feedstuff.

## Patentansprüche

1. Verfahren zum Herstellen eines Nahrungszusatzstoffes zur Verwendung für die Prävention von Darmkrankheiten, wobei in dem Verfahren Bierhefe durch einen hydrolytischen Prozess behandelt wird, **dadurch gekennzeichnet, dass** Bierhefe filtriert wird und die filtrierte Bierhefe hydrolytisch behandelt wird, so dass die zelluläre Struktur aufbricht und die effektive Konzentration von Oligo- und/oder Polysacchariden, Betaglucan und/oder Proteinen auf der Oberfläche der zellulären Strukturen erhöht wird.

2. Verfahren nach Anspruch 1, **dadurch gekennzeichnet, dass** die Bierhefe mechanisch filtriert wird.

3. Verfahren nach Anspruch 1 oder 2, **dadurch gekennzeichnet, dass** die Bierhefe auf einen Trockenmassemindestgehalt von 15%, bevorzugt zu einem Trockenmassegehalt von 18 - 20% filtriert wird.

4. Verfahren nach einem der Ansprüche 1 - 3, **dadurch gekennzeichnet, dass** die filtrierte Bierhefe mit einer Säure und/oder einer Base behandelt wird.

5. Verfahren nach einem der Ansprüche 1 - 4, **dadurch gekennzeichnet, dass** die filtrierte Bierhefe enzymatisch behandelt wird.

6. Verfahren nach einem der Ansprüche 1 - 5, **dadurch gekennzeichnet, dass** die filtrierte Bierhefe mit Wärme und/oder mechanisch, hydrostatisch oder pneumatisch behandelt wird, so dass die zelluläre Struktur abgebaut wird.

7. Verfahren nach einem der Ansprüche 1 - 6, **dadurch gekennzeichnet, dass** das erhaltene Produkt als solches ohne Fraktionierung verwendet wird.

8. Verfahren nach einem der Ansprüche 1-7, **dadurch gekennzeichnet, dass** das Oligo- und/oder Polysaccharid, das Betaglucan und/oder das erhaltene Proteinprodukt zu Nahrung in einer Menge von 0,01 - 1,0 Gew.-%, bevorzugt 0,1 - 0,3 Gew.-%, berechnet bezüglich der Trockenmasse, zugegeben wird.

9. Nahrungszusatzstoff zur Prävention von Darmkrankheiten, **dadurch gekennzeichnet, dass** der Zusatzstoff hergestellt wurde durch Behandeln filtrierter Bierhefe durch einen hydrolytischen Prozess, so dass die zelluläre Struktur aufbricht und die effektive Konzentration von Oligo- und/oder Polysacchariden, Betaglucan und/oder Proteinen auf der Oberfläche der zellulären Struktur erhöht wird.

10. Zusatzstoff nach Anspruch 9, **dadurch gekennzeichnet, dass** der Zusatzstoff durch mechanisches Filtrieren von Bierhefe hergestellt wurde.

11. Zusatzstoff nach Anspruch 9 oder 10, **dadurch gekennzeichnet, dass** der Zusatzstoff durch Behandeln des Rohmaterials mit einer Säure und/oder einer Base hergestellt wurde.

12. Zusatzstoff nach einem der Ansprüche 9 - 11, **dadurch gekennzeichnet, dass** der Zusatzstoff durch enzymatisches Behandeln des Rohmaterials hergestellt wurde.

13. Zusatzstoff nach einem der Ansprüche 9 - 12, **dadurch gekennzeichnet, dass** die Bierhefe mit Wärme und/oder mechanisch, hydrostatisch oder pneumatisch behandelt wurde, so dass die zelluläre Struktur abgebaut wurde.

14. Verwendung eines Nahrungszusatzstoffes wie in einem der Ansprüche 9-13 definiert, zur Zubereitung von Nahrungsmittel, die für die Prävention von Darmkrankheiten vorgesehen sind, wobei die Menge des verwendeten Zusatzstoffes 0,01 - 1,0 Gew.-%, bevorzugt 0,1 - 0,3 Gew.-%, gemischt in Nährstoffen beträgt.

15. Zubereitung, die einen Nahrungszusatzstoff enthält und die dafür vorgesehen ist, einem zu fütternden Lebewesen gegeben zu werden, **dadurch gekennzeichnet, dass** die Zubereitung ein Produkt wie in einem der Ansprüche 10 - 13 definiert, in einer Menge von 0,01 - 1,0 Gew.-%, bevorzugt 0,1 - 0,3 Gew.-%, der täglichen Portion von Nahrungsmitteln und/oder Futtermitteln enthält.

## Revendications

1. Procédé de production d'un additif alimentaire à utiliser pour la prévention de maladies intestinales, ledit procédé comprenant le traitement de levure de bière par un procédé hydrolytique, **caractérisé en ce que** la levure de bière est filtrée et la levure de bière filtrée est traitée de façon hydrolytique de sorte que la structure cellulaire s'ouvre et que la concentration efficace d'oligo- et/ou de polysaccharides, de bêtaglucane et/ou de protéines sur la surface des structures cellulaires soit accrue.

2. Procédé selon la revendication 1, **caractérisé en ce que** la levure de bière est filtrée mécaniquement.

3. Procédé selon la revendication 1 ou 2, **caractérisé en ce que** la levure de bière est filtrée jusqu'à une teneur minimale en matière sèche de 15 %, de préférence, jusqu'à une teneur en matière sèche de 18 à 20 %.

4. Procédé selon l'une quelconque des revendications 1 à 3, **caractérisé en ce que** la levure de bière filtrée est traitée avec un acide et/ou un alcali.

5. Procédé selon l'une quelconque des revendications 1 à 4, **caractérisé en ce que** la levure de bière filtrée est traitée de façon enzymatique.

6. Procédé selon l'une quelconque des revendications 1 à 5, **caractérisé en ce que** la levure de bière filtrée est traitée thermiquement et/ou mécaniquement, de façon hydrostatique ou pneumatique, de sorte que la structure cellulaire soit décomposée.

7. Procédé selon l'une quelconque des revendications 1 à 6, **caractérisé en ce que** le produit obtenu est utilisé tel quel sans fractionnement.

8. Procédé selon l'une quelconque des revendications 1 à 7, **caractérisé en ce que** l'oligo-et/ou le polysaccharide, le bêtaglucane et/ou le produit protéique obtenu est ajouté au produit alimentaire en une quantité de 0,01 à 1,0 % en poids, de préférence, de 0,1 à 0,3 % en poids, calculé en termes de matière sèche.

9. Additif alimentaire pour la prévention des maladies intestinales, **caractérisé en ce que** l'additif a été produit par traitement de la levure de bière filtrée par un procédé hydrolytique de sorte que la structure cellulaire s'ouvre et que la concentration en oligo- et/ou polysaccharides, en bêtaglucane et/ou en protéines sur la surface des structures cellulaires soit accrue.

10. Additif selon la revendication 9, **caractérisé en ce que** l'additif a été produit par filtrage mécanique de la levure de bière.

11. Additif selon la revendication 9 ou 10, **caractérisé en ce que** l'additif a été produit par traitement du matériau brut avec un acide et/ou un alcali.

12. Additif selon l'une quelconque des revendications 9 à 11, **caractérisé en ce que** l'additif a été produit par traitement enzymatique du matériau brut.

13. Additif selon l'une quelconque des revendications 9 à 12, **caractérisé en ce que** la levure de bière a été traitée thermiquement et/ou mécaniquement, de façon hydrostatique ou pneumatique, de sorte que la structure cellulaire soit décomposée.

14. Utilisation d'un additif alimentaire tel que défini dans l'une quelconque des revendications 9 à 13, pour la préparation d'un produit alimentaire destiné à la prévention des maladies intestinales, la quantité de l'additif utilisé étant de 0,01 à 1,0 % en poids, de préférence de 0,1 à 0,3 % en poids, mélangée dans des substances nutritives.

15. Préparation contenant un additif alimentaire, et destiné à être administré à une créature devant être alimentée, **caractérisée en ce que** la préparation contient un produit tel que défini dans l'une quelconque des revendications 10 à 13, en une quantité de 0,01 à 1,0 % en poids, de préférence, de 0,1 à 0,3% en poids de la part quotidienne d'aliments et/ou d'aliments pour animaux.
